# EUROPEAN PATENT APPLICATION

(11) **EP 4 406 528 A1**
(43) Date of publication of application: **31.07.2024**
(21) Application number: 22872940.6
(22) Date of filing: 21.09.2022
(51) Int. Cl.: A61K 8/19, A61Q 5/00, A61Q 19/00, A61M 11/00, A45D 44/00, A61H 33/12

(54) **AEROSOLIZED AGENT FOR SKIN OR HAIR, COSMETIC TREATMENT METHOD, AND SPRAYING DEVICE**

(30) Priority: 21.09.2021 JP 2021152888; 21.09.2021 JP 2021152889; 07.10.2021 JP 2021165726
(71) Applicant: AISIN CORPORATION, Kariya-shi, Aichi 448-8650 (JP)
(72) Inventor: HIRANO Akiyoshi, Kariya-shi, Aichi 448-8650 (JP); INOUE Shinsuke, Kariya-shi, Aichi 448-8650 (JP); SHIGEMORI Yasushi, Kariya-shi, Aichi 448-8650 (JP); TABATA Yuki, Kariya-shi, Aichi 448-8650 (JP)
(74) Representative: Cabinet Beau de Loménie
(86) International application number: PCT/JP2022/035195
(87) International publication number: WO 2023/048189

(57) **Abstract**

A cosmetic treatment method includes: spraying, toward skin or hair: an atomized medicinal agent in which a medicinal agent containing a predetermined active ingredient is atomized; and fine water that is uncharged and has a particle size of less than or equal to 50 nanometers. Therefore, the sprayed fine water enters the skin or hair to contribute to maintenance of moisturization, and at the same time, forms a route through which the active ingredient permeates, thereby promoting the permeation of the active ingredient; therefore, the permeability of the active ingredient can be more appropriately improved.

## Description

### TECHNICAL FIELD

The present disclosure relates to a spray agent for skin or hair, a cosmetic treatment method, and a spray device.

### BACKGROUND ART

Conventionally, there has been proposed a device including: a liquid agent spray mechanism that electrostatically atomizes and sprays a liquid agent; and a mist generation mechanism that generates mist, and the device sprays, together with the mist, the liquid agent whose particle size has been reduced by electrostatic atomization. For example, in the device of Patent Literature 1, a liquid agent spray port of a liquid agent spray mechanism and a mist discharge port of a mist generation mechanism are separately provided, and the liquid agent spray mechanism and the mist generation mechanism are controlled so as to spray simultaneously.

### CITATIONS LIST

### PATENT LITERATURE

Patent Literature 1: JP 2010-172659 A

### SUMMARY OF INVENTION

### TECHNICAL PROBLEMS

However, when a liquid agent and water particles are charged by electrostatic atomization or the like as in the case of the above-described device, the liquid agent and the water particles are electrically sucked by a surface of a horny layer of the skin or the like and rather hardly permeates into the inside of the horny layer in some cases. In addition, in the case of a device in which the liquid agent spray port and the mist discharge port are separately provided, it may be difficult to uniformly mix the sprayed liquid agent and the mist.

The present disclosure is to more appropriately improve the permeability of an active ingredient when a spray agent is sprayed to skin or hair.

### SOLUTIONS TO PROBLEMS

The present disclosure has adopted the following means to achieve the above product, method, and device.

A spray agent of the present disclosure is a spray agent for skin or hair comprises a mixture, wherein the mixture includes: an atomized medicinal agent in which a medicinal agent containing a predetermined active ingredient is atomized; and fine water that is uncharged and has a particle size of less than or equal to 50 nanometers.

The spray agent of the present disclosure is a mixture of an atomized medicinal agent and fine water that is uncharged and has a particle size of less than or equal to 50 nanometers. When the spray agent is sprayed to skin or hair, the fine water enters the skin or hair to contribute to maintenance of moisturization, and at the same time, forms a route through which the active ingredient permeates, thereby promoting the permeation of the active ingredient; therefore, the permeability of the active ingredient can be more appropriately improved. In addition, since the fine water is uncharged particles, that is, is not charged, the fine water is not electrically sucked to a surface of a horny layer of the skin or the like and can permeate into the horny layer. Furthermore, it is said that gaps in the horny layer of the skin are about 20 to 50 nanometers or less, and the fine water has a particle size of less than or equal to 50 nanometers, so that the fine water can easily enter the gaps in the horny layer, and this fact is also a reason for the high permeation effect.

A cosmetic treatment method of the present disclosure comprises spraying toward skin or hair, an atomized medicinal agent in which a medicinal agent containing a predetermined active ingredient is atomized, and fine water that is uncharged and has a particle size of less than or equal to 50 nanometers. Therefore, it is possible to more appropriately improve the permeability of the active ingredient when the spray agent is sprayed to the skin or hair.

The cosmetic treatment method of the present disclosure may comprise: mixing the atomized medicinal agent and the fine water, wherein, in the step of spraying, the spray agent in which the atomized medicinal agent and the fine water are mixed in the step of mixing is sprayed toward skin or hair. By this method, it possible to spray the atomized medicinal agent and the fine water in a state where the atomized medicinal agent and the fine water are sufficiently mixed as compared with the case of separately spraying the atomized medicinal agent and the fine water, so that uneven spraying can be prevented to further appropriately improve the permeability of the active ingredient. Furthermore, mixing amounts (ratio) of the atomized medicinal agent and the fine water can be easily adjusted.

A spray device of the present disclosure comprises: an atomizer that generates an atomized medicinal agent in which a medicinal agent containing a predetermined active ingredient is atomized; a fine water generator that changes to a moisture absorption state in which moisture in air is made to be adsorbed to a conductive polymer film due to a decrease in temperature, and to a moisture release state in which the moisture adsorbed to the conductive polymer film is made to be released as fine water having a particle size of less than or equal to 50 nanometers due to an increase in temperature; and a spray unit that sprays the atomized medicinal agent and the fine water toward skin or hair.

The spray device of the present disclosure sprays, from the spray unit toward skin or hair, the atomized medicinal agent generated by the atomizer, and the fine water released by changing the fine water generator to the moisture absorption state and the moisture release state. Therefore, it is possible to more appropriately improve the permeability of the active ingredient when the spray agent is sprayed to the skin or hair.

The spray device of the present disclosure may be configured as follows. The spray device further includes a mixing unit that communicates with the atomizer, the fine water generator, and the spray unit and mixes the atomized medicinal agent and the fine water. The spray unit sprays a spray agent in which the atomized medicinal agent and the fine water are mixed in the mixing unit. This configuration makes it possible to adjust a mixing ratio between the atomized medicinal agent and the fine water as compared with the case of separately spraying the atomized medicinal agent and the fine water, so that uneven spraying can be prevented, so that the permeability of the active ingredient can be further appropriately improved.

Furthermore, the spray device may be configured as follows. The spray device includes: a mixing unit that communicates with the atomizer, the fine water generator, and the spray unit and mixes the atomized medicinal agent and the fine water; and a control unit controls so as to increase a concentration of the fine water in the mixing unit by repeating the following states: a moisture absorption state in which an inside of the mixing unit is sealed and air outside the mixing unit is supplied to a conductive polymer film whose temperature is reduced so as to cause moisture in the air to be adsorbed to the conductive polymer film; and a moisture release state in which air in the mixing unit is circulated to the conductive polymer film whose temperature is increased so as to cause the moisture adsorbed to the conductive polymer film to be released, into the mixing unit, to be fine water having a particle size of less than or equal to 50 nanometers or less. The spray unit sprays the spray agent in which the atomized medicinal agent and the fine water are mixed in the mixing unit. This configuration makes it possible to mix the fine water and the atomized medicinal agent in a state where the concentration of the fine water in the mixing unit is increased. Therefore, the spray agent in which the fine water is sufficiently mixed can be sprayed, so that the permeability of the active ingredient can be further improved while enhancing a moisturizing effect.

The spray device of the present disclosure may be configured as follows. The spray device further comprises a switching part that selects any one of a sealed state in which communication between the fine water generator and the mixing unit is blocked and the air outside the mixing unit is supplied to the fine water generator, and a circulation state in which communication is established between the fine water generator and the mixing unit and air inside the mixing unit is supplied to the fine water generator. The fine water generator enters the moisture absorption state when the switching part in the sealed state, and the moisture release state when the switching part is in the circulation state, such that a concentration of the fine water in the mixing unit is increased. This configuration makes it possible to bring the inside of the mixing unit in a high concentration environment of fine water and to cause the fine water to be sufficiently mixed with the spray agent.

The cosmetic treatment method of the present disclosure may be as follows. The cosmetic treatment method includes: adsorbing moisture to a conductive polymer film having a nanochannel; and increasing a temperature of the conductive polymer film to release the moisture adsorbed to the conductive polymer film. In the step of mixing, the fine water is mixed with the atomized medicinal agent. This method makes it possible to cause the fine water having a smaller particle size to be contained in the atomized medicinal agent, so that the permeability of the active ingredient can be further improved.

### BRIEF DESCRIPTION OF DRAWINGS

FIG. 1 is a configuration diagram schematically illustrating configuration of a cosmetic treatment device 1.
FIG. 2 is a configuration diagram schematically illustrating a configuration of a spray device 10.
FIG. 3A is a configuration diagram schematically illustrating a configuration of a fine water generation cartridge 30, and FIG. 3B is a configuration diagram schematically illustrating a configuration of a fine water release element 34.
FIG. 4 is a process chart illustrating an example of a cosmetic treatment method.
FIG. 5 is a flowchart illustrating an example of a spray process.
FIG. 6 is an explanatory view illustrating an image in which fine water permeates into skin.
FIG. 7 is a configuration diagram schematically illustrating a configuration of a spray device 10B of a variation.
FIG. 8 is a configuration diagram schematically illustrating a configuration of a spray device 100 of a second embodiment.
FIG. 9 is a configuration diagram schematically illustrating a configuration of a spray device 200 of a third embodiment.

### DESCRIPTION OF EMBODIMENTS

### First embodiment

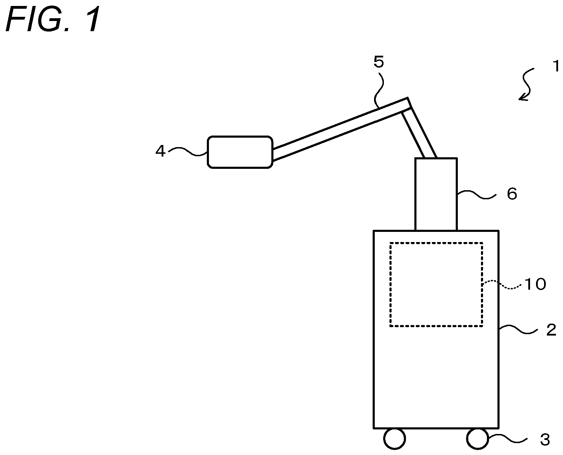

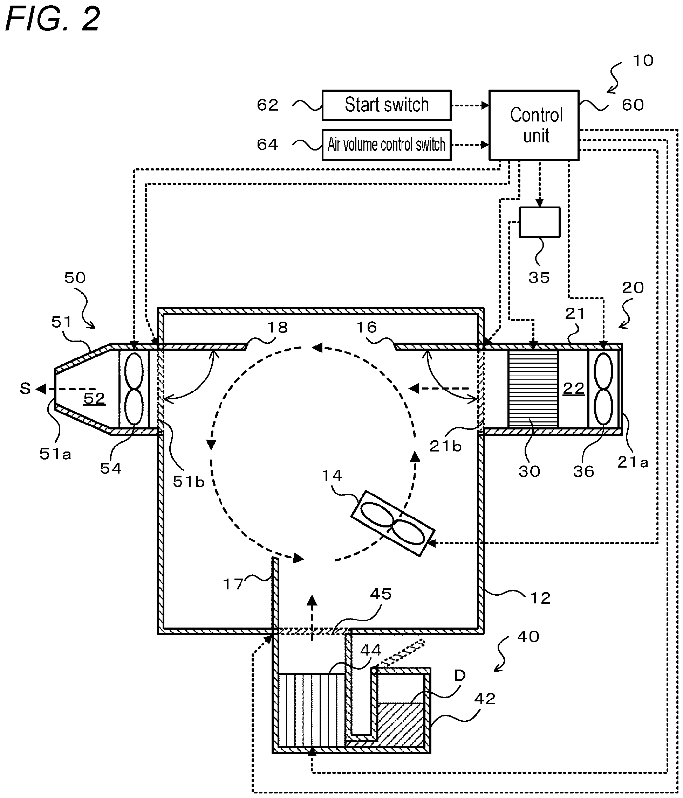

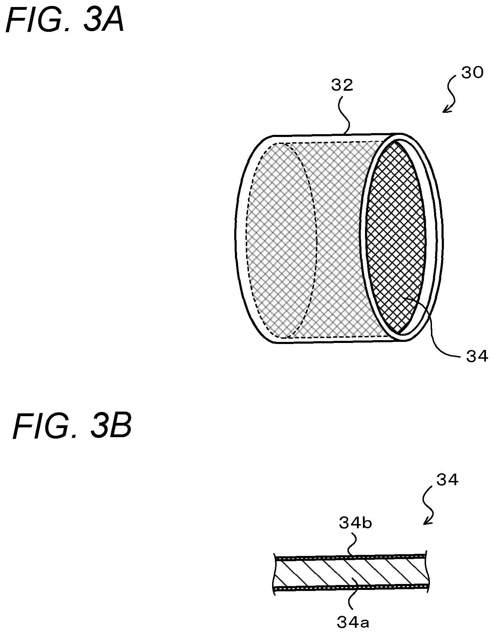

Next, a first embodiment of the present disclosure will be described with reference to the drawings. FIG. 1 is a configuration diagram schematically illustrating a configuration of a cosmetic treatment device 1, FIG. 2 is a configuration diagram schematically illustrating a configuration of a spray device 10, FIG. 3A is a configuration diagram schematically illustrating a configuration of a fine water generation cartridge 30, and FIG. 3B is a configuration diagram schematically illustrating a configuration of a fine water release element 34. FIG. 3 is a configuration diagram schematically illustrating a configuration of a fine water generation cartridge 30.

As shown in Fig. 1, the cosmetic treatment device 1 includes: a device main body 2 that houses a spray device 10; a discharge head 4 that discharges a spray agent generated by the spray device 10; a movable arm 5 having a distal end to which the discharge head 4 is attached; and a support part 6 that supports a proximal end of the movable arm 5 on the device main body 2. The device main body 2 is movably supported by casters 3 attached to the bottom. The movable arm 5 has a supply passage formed inside the arm for the spray agent to be supplied to the discharge head 4. The discharge head 4 is turnably attached to the distal end of the movable arm 5, and a proximal end of the movable arm 5 is turnably supported by the support part 6. Therefore, the discharge head 4 can move and turn in the vertical, horizontal, and front-back directions with respect to the device main body 2, and can be adjusted to a position suitable for an object to be treated. The cosmetic treatment device 1 is placed in, for example, an aesthetic salon and a beauty salon, and may be placed in an individual's home.

As illustrated in FIG. 2, the spray device 10 includes a mixing tank 12, a fine water supply unit 20, a medicinal agent atomization unit 40, and a spray unit 50.

The mixing tank 12 is formed in a rectangular parallelepiped shape or a tubular shape, includes a mixing fan 14 in the inside thereof, and, to the mixing tank 12 there are attached the fine water supply unit 20, the medicinal agent atomization unit 40, and the spray unit 50 to be communicable with the inside of the tank. The mixing fan 14 is disposed so as to generate a swirl flow in the mixing tank 12 by blowing air. The mixing tank 12 further includes: a first opening and closing damper 16 that switches between communication and blocking between the fine water supply unit 20 and the inside of the tank by an opening and closing operation; a second opening and closing damper 17 that switches between communication and blocking between the medicinal agent atomization unit 40 and the inside of the tank by an opening and closing operation; and a third opening and closing damper 18 that switches between communication and blocking between the spray unit 50 and the inside of the tank by an opening and closing operation. Each of the first to third opening and closing dampers 16 to 18 causes, by driving of a motor (not illustrated), an opening and closing plate to operate to perform an opening and closing operation.

The fine water supply unit 20 includes: a duct 21 in which an air passage 22 is formed; the fine water generation cartridge 30; an energization circuit 35; and a supply fan 36.

The duct 21 is a tubular member whose both ends are opened, and has a communication port 21b that communicates an air intake port 21a opened outside the mixing tank 12 and the mixing tank 12 and that is opened and closed by the first opening and closing damper 16, thereby forming a linear air passage 22. The fine water generation cartridge 30 and the supply fan 36 are disposed in the air passage 22 in the order of the supply fan 36 and the fine water generation cartridge 30 from the air intake port 21a side.

As illustrated in FIG. 3A, the fine water generation cartridge 30 includes: a case 32 in a cylindrical shape having such an outer diameter that the case can be disposed in the air passage 22; and a fine water generation element 34 provided in the case 32. As shown in FIG. 3B, the fine water generation element 34 includes a base material 34a and a conductive polymer film 34b formed on the surface of the base material 34a.

The base material 34a is formed of a conductive material such as a metal material including a stainless steel-based metal or a copper-based metal, a carbon material, or a conductive ceramic material. In the present embodiment, a metal foil of stainless steel to which aluminum is added is used. The fine water generation element 34 is formed in a corrugated plate shape, a honeycomb shape, a spiral shape, or the like so that air can flow through and a surface area of the base material 34a (conductive polymer film 34b) is as large as possible. An energization circuit 35 including a power supply and a switch is connected to the base material 34a. When the switch is turned on by the control unit 60, the energization circuit 35 enters an energization state for energizing the base material 34a, and when the switch is turned off by the control unit 60, the energization circuit 35 enters a non-energization state for cutting off energization to the base material 34a.

The conductive polymer film 34b is formed of a polymer compound having conductivity such as a thiophene-based conductive polymer. In the present embodiment, the conductive polymer 34b is formed of PEDOT/PSS (poly (3,4-ethylenedioxythiophene)/poly (styrenesulfonic acid)) among thiophene-based conductive polymers. PEDOT/PSS has a structure in which PEDOT is dispersed in PSS having a sulfonic acid group, which is a hydrogen bondable acidic functional group. In addition, nanochannels which are channels having a nanometer size of about 2 nanometers (nm) are formed at boundary portions between PEDOT and PSS. Because a large amount of a sulfonic acid group is present in the nanochannels, moisture present on the surface of conductive polymer film 34b moves inside along the sulfonic acid group in the nanochannels due to a concentration difference between the surface and the inside when the amount of moisture on the surface is large and the amount of moisture in the inside is small. Consequently, the conductive polymer film 34b adsorbs moisture. In addition, when the amount of moisture on the surface is small and the amount of moisture inside is large in a state where the moisture is adsorbed inside, the moisture moves to the surface along the sulfonic acid group in the nanochannels due to the concentration difference between the surface and the inside. Consequently, moisture is released as fine water from the conductive polymer film 34b. Furthermore, in a state where the temperature of the conductive polymer film 34b has increased, rapid release of moisture (fine water) is promoted as compared with a case where the moisture moves only by the concentration difference, and in a state where the temperature of conductive polymer film 34b has decreased, rapid adsorption of moisture is promoted as compared with a case where the moisture moves only by the concentration difference. As described above, the fine water generation cartridge 30 (fine water generation element 34) changes to a moisture absorption state where moisture in the air is adsorbed to the conductive polymer film 34b due to a decrease in temperature, and changes to a moisture release state where the adsorbed moisture is released from the conductive polymer film 34b due to an increase in temperature. Note that a thickness of the conductive polymer film 34b can be appropriately determined in accordance with a required adsorption amount (release amount) of the fine water. For example, when conductive polymer film 34b is formed to have a thickness of, for example, 1 to 30 micrometers, moisture sufficient to release fine water can be adsorbed to the conductive polymer film 34b in about several seconds to several tens of seconds.

In addition, the fine water generation cartridge 30 releases, from the conductive polymer film 34b of the fine water generation element 34, uncharged fine water having a water particle size of 50 nanometers or less, for example, a particle size of about 1 to 2 nanometers. The reason that the particles have such a particle size is considered as follows. Since the size of the nanochannels is 2 nanometers or less, the increase in the temperature of the conductive polymer film increases the mobility and a pressure of water in the nanochannels, so that the moisture jumps out from the nanochannels. In addition, even when the water particles aggregate after jumping out, the particle size of the aggregation is distributed in the range of 50 nanometers or less. A detailed description about such generation of fine water in the fine water generation cartridge 30 (conductive polymer film 34b) is described in WO 2020/054100 A, JP 2019-018195 A, and the like of the applicant of the present application; therefore, a further detailed description is omitted.

The supply fan 36 blows air from the air intake port 21a outside the mixing tank 12 toward the communication port 21b inside the mixing tank 12 by rotational driving in a predetermined rotation direction. Therefore, the air sucked into the air passage 22 can be sent into the mixing tank 12 through the fine water generation cartridge 30. The supply fan 36 is rotationally driven by a motor (not illustrated), and is controlled by the control unit 60 by pulse width modulation (PWM) control, voltage control, or the like. Note that the supply fan 36 may be a propeller fan, a sirocco fan, or the like.

The medicinal agent atomization unit 40 includes: a medicinal agent tank 42 that contains a medicinal agent D; and an atomizer 44 that atomizes the medicinal agent D contained in the medicinal agent tank 42 and supplies the medicinal agent D from a supply port 45 into the mixing tank 12. The atomizer 44 is configured to atomize the medicinal agent D by, for example, an ultrasonic atomization method in which the medicinal agent D is atomized by vibration of a piezoelectric element. The atomizer 44 may be configured to atomize the medicinal agent D by another method such as an air spray method, an electrostatic atomization method, or a centrifugal atomization method. Furthermore, the supply port 45 is opened and closed by the second opening and closing damper 17.

Furthermore, the medicinal agent D contained in the medicinal agent tank 42 is mixed with a cosmetic component, a medicinal component, or the like as a predetermined active ingredient. Examples of the medicinal agent D and the active ingredient include, without being limited thereto: moisturizing components, herbal extracts, enzymes such as tyrosinase, superoxide dismutase, and lipase; vitamins and derivatives thereof such as retinol, ascorbic acid, tocopherol, pyridoxal, and riboflavin; organic dyes such as 0-carotene and chlorophyll; moisture components such as glycerin, sorbitol, urea, lactic acid, propylene glycol, polyethylene glycol, and copolymers, and glucose derivatives; emollient components such as paraffin, stearyl alcohol, cetyl alcohol, squalene, silicone oil, and stearyl; treatment components; dandruff inhibition components; pilatory and hair growth components; and the like.

The spray unit 50 includes: a duct 51 in which an air passage 52 is formed; and a spray fan 54. The duct 51 is a tubular member whose both ends are opened, and has a communication port 51b that communicates a spray port 51a opened outside the mixing tank 12 with the mixing tank 12 and that is opened and closed by the third opening and closing damper 18. Furthermore, the duct 51 is formed in a tapered shape such that, on the spray port 51a side with respect to the spray fan 54, a cross-sectional area of the air passage 52 is smaller toward the spray port 51a side. The spray unit 50 takes in the spray agent in the mixing tank 12 and sprays the spray agent from the spray port 51a by operation of the spray fan 54. The spray agent sprayed from the spray port 51a reaches the discharge head 4 through the supply passage in the movable arm 5.

The control unit 60 is configured as a microprocessor mainly configured with a CPU, and includes a ROM, a RAM, and input and output ports in addition to the CPU. To the control unit 60 there are input, via an input port: an operation signal from a start switch 62 to start the operation of the spray device 10; an operation signal from an air volume control switch 64 to individually or collectively control air volumes of respective ones of the fans 14, 36, and 54; and other signals. Furthermore, from the control unit 60 there are output, via an output port: drive signals to the motors for rotationally driving respective ones of the fans 14, 36, and 54; drive signals to the first to third opening and closing dampers 16 to 18; a drive signal to the switch of the energization circuit 35; and other signals.

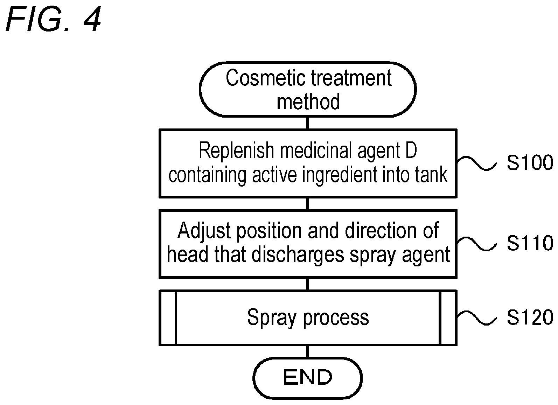

Next, a cosmetic treatment method of spraying the spray agent to skin or hair by using the cosmetic treatment device 1 (spray device 10) configured as described above will be described. FIG. 4 is a process chart illustrating an example of the cosmetic treatment method. A practitioner (store clerk) of an aesthetic salon or the like replenishes (stores) the medicinal agent D containing an active ingredient into the medicinal agent tank 42 of the medicinal agent atomization unit 40 (step S100). Next, the practitioner adjusts the position and direction of the discharge head 4 so that the discharge head 4 can discharge the spray agent toward a spray target portion of a person to be treated (step S110), and operates the start switch 62 to perform a spray process by the spray device 10 (step S110). By this spray process, the spray agent generated by the spray device 10 is discharged from the discharge head 4 and sprayed to the skin or hair of the person to be treated.

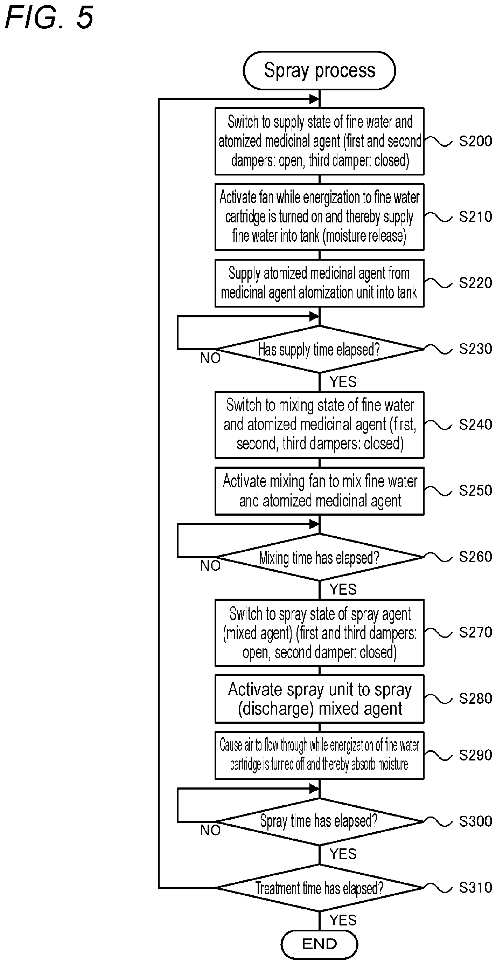

FIG. 5 is a process chart illustrating an example of the spray process. In the spray process, the control unit 60 of the spray device 10 switches to a supply state of the fine water and the atomized medicinal agent by bringing the first opening and closing damper 16 and the second opening and closing damper 17 into an open state and bringing the third opening and closing damper 18 into a closed state (S200). In this supply state, the fine water is supplied into the mixing tank 12 by a moisture release control in which energization to the fine water generation cartridge 30 is turned on and the supply fan 36 is driven in a predetermined rotation direction (S210), the medicinal agent atomization unit 40 is operated to supply the atomized medicinal agent into the mixing tank 12 (S220), and the process waits for a predetermined supply time to elapse (S230). In S210, since the supply fan 36 is rotationally driven in the predetermined rotation direction, the air sucked from the air intake port 21a is discharged from the communication port 21b into the mixing tank 12 (see the dotted arrow in FIG. 1). Furthermore, since energization to the fine water generation cartridge 30 is turned on, the temperature of the conductive polymer film 34b increases, thereby promoting the release of the fine water. Note that if the spray process is started in a state where moisture is not adsorbed to the conductive polymer film 34b, moisture absorption control in which moisture is adsorbed to the conductive polymer film 34b may be performed before S210 by driving the supply fan 36 while energization to the fine water generation cartridge 30 is turned off.

If the control unit 60 determines, in S230, that the supply time has elapsed, the control unit 60 switches to a mixing state of the fine water and the atomized medicinal agent, by bringing all of the first opening and closing damper 16, the second opening and closing damper 17, and the third opening and closing damper 18 into the closed state (S240). By activating the mixing fan 14 in this mixing state, the fine water and the atomized medicinal agent are mixed in the mixing tank 12 (S250), and a predetermined mixing time is waited (S260). By the operation of the mixing fan 14, an air flow is generated in the mixing tank 12 (see the dotted arrows in FIG. 2), thereby promoting mixing, so that the fine water and the atomized medicinal agent can be uniformly mixed.

Furthermore, if the control unit 60 determines, in S260, that the mixing time has elapsed, the control unit 60 switches to a spray state of the spray agent (mixed agent) S in which the fine water and the atomized medicinal agent are mixed, by bringing the first opening and closing damper 16 and the third opening and closing damper 18 into the open state and bringing the second opening and closing damper 17 into the closed state (S270). In this spray state, it is waited for a predetermined spray time to elapse (S300) while the spray fan 54 is operated to spray the spray agent (mixed agent) in the mixing tank 12 from the spray unit 50 (S280) and while performing the moisture absorption control in which moisture is adsorbed to the conductive polymer film 34b with energization to the fine water generation cartridge 30 turned off (S290). Note that the operation of the spray fan 54 causes the air sucked into the air passage 22 from the air intake port 21a to pass through the fine water generation cartridge 30, so that the temperature of the conductive polymer film 34b decreases, thereby promoting the adsorption of moisture.

If the control unit 60 determines, in S300, that the spray time has elapsed, the control unit 60 determines if a predetermined treatment time has elapsed (S310), and it is determined that the treatment time has not elapsed, the flow returns to S200 to perform the process. In this manner, the control unit 60 sequentially and repeatedly performs the following: the supply of the fine water and the spray agent; the mixing of the fine water and the atomized medicinal agent; and the spraying of the spray agent S. The supply time, mixing time, and spray time can be appropriately set to about several 10 seconds to several minutes depending the following: a moisture absorption capacity (moisture release capacity) of the fine water generation cartridge 30; an atomizing capacity of the medicinal agent atomization unit 40; mixing amounts (ratio) of the fine water and the atomized medicinal agent; an spraying capacity of the spray unit 50; a size of the mixing tank 12; a type and amount of the medicinal agent D; a contained active ingredient; and the like. Similarly, the treatment time can be appropriately set to ten and several minutes or several tens of minutes.

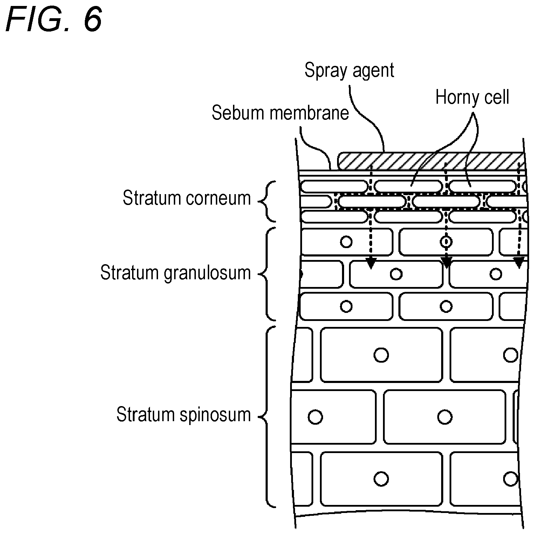

Here, FIG. 6 is an explanatory view illustrating an image in which the fine water permeates into skin. As illustrated, an epidermis of skin includes a stratum corneum covered with a sebum membrane, a stratum granulosum, a stratum spinosum, a basal layer (not illustrated), and the like. When the spray agent S is sprayed to the skin, the fine water enters the stratum corneum and enters the intercellular lipids between the horny cells (see the arrows in FIG. 6). The horny surface has gaps of about 20 to 50 nanometers or less. Since the fine water has a particle size of 50 nanometers or less and about 1 to 2 nanometers, the fine water easily enters into the stratum corneum. Furthermore, because the skin is generally positively charged, if the water particles are negatively charged, the water particles are electrically attracted by the skin surface and hardly enter into the stratum corneum, and if the water particles are positively charged, the water particles are repelled and hardly reach the skin surface. Because the fine water is not charged, such situations do not occur. The fine water that has thus entered into the stratum corneum forms routes of water along the intercellular lipids around the horny cells. At that time, the moisture amount of the intercellular lipids is improved, and the moisture also enters into the natural moisturizing component in the horny cells; therefore, the moisture amount of the entire stratum corneum can be brought close to an appropriate value, thereby improving the moisture retaining property. In addition, the active ingredient of the spray agent S passes through the routes of water formed, by the fine water, in the intercellular lipids and diffuses in the skin; therefore, permeation of the active ingredient can be promoted. Note that, in addition to the above-described pattern in which the routes of water are formed of fine water and the medicinal agent is diffused through the routes, there can be considered a pattern of permeation of medicinal agent, in which pattern the medicinal agent is pushed in together with the fine water by the permeation force of the fine water at the same time as the fine water permeates. As described above, the fine water provides the effect of improving the moisture retaining property due to the supply of moisture, and provides synergistically provides the effect of improving the permeability of the active ingredient accompanying the formation of the routes of water formed of fine water or provides the effect of improving the permeability of the active ingredient due to the permeation force of the fine water. How the fine water permeates into the skin is described above. However, the following pattern of permeation of medicinal agent can also be considered. The fine water similarly permeates into and remains in the fine gaps (about 50 nm or less) between cuticles on the hair surface to moisturize the hair, and water routes are formed of fine water. The medicinal agent diffuses through the water routes, and, in addition, the medicinal agent is pushed in together with the fine water by the permeation force of the fine water.

The spray agent S is a mixture of an atomized medicinal agent and fine water having a particle size of less than or equal to 50 nanometers. Therefore, when the spray agent S is sprayed to skin or hair, the fine water permeates, so that routes of water through which the active ingredient permeates can be formed and so that moisturization can be sufficiently performed. Therefore, it is possible to enhance the moisturizing effect and the permeation effect.

Further, in the cosmetic treatment method, since the spray agent S in which the atomized medicinal agent and the fine water are mixed is sprayed toward skin or hair, it is possible to suppress uneven spray (adhesion) of the atomized medicinal agent and the fine water as compared with a case where the atomized medicinal agent and the fine water are separately sprayed; therefore, it is possible to more appropriately improve the permeability and the moisture retaining property of the active ingredient. Furthermore, with the spray device 10, mixing amounts (ratio) of the atomized medicinal agent and the fine water can be easily adjusted in the mixing tank 12.

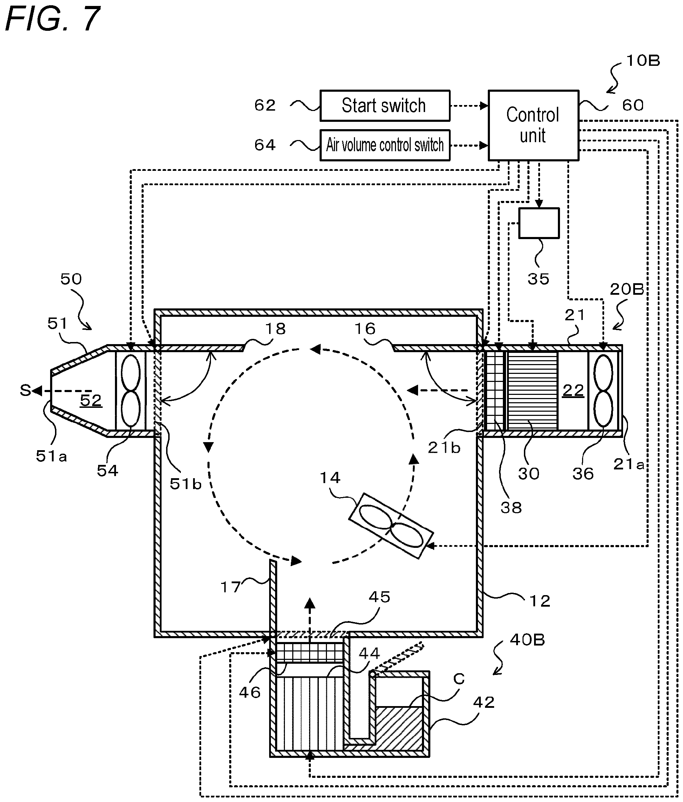

Here, the spray device 10 of the first embodiment may be configured as follows. FIG. 7 is a configuration diagram schematically illustrating a configuration of a spray device 10B of a variation. The spray device 10B is configured similarly to the first embodiment except that the configurations of the fine water supply unit 20B and the medicinal agent atomization unit 40B are different from those of the first embodiment. The fine water supply unit 20B includes the discharge element 38 in the duct 21 on the communication port 21b side (mixing tank 12 side) with respect to the fine water generation cartridge 30. Furthermore, the medicinal agent atomization unit 40B includes a discharge element 46 on the near side (atomizer 44 side) of the supply port 45. Each of the discharge elements 38 and 46 is configured to include, for example, a first electrode (discharge electrode) and a second electrode (opposite electrode) disposed opposite to the first electrode and to perform plasma discharge on the basis of a drive signal from the control unit 60.

With the spray device 10B, by causing the fine water discharged from the fine water generation cartridge 30 and the atomized medicinal agent discharged from the medicinal agent atomization unit 40B to pass through plasma regions generated by the discharges, the fine water and the atomized medicinal agent can be charged and then supplied to the mixing tank 12. This makes it possible to deodorize odorous components contained in outside air passing through the fine water generation cartridge 30 and the medicinal agent atomization unit 40B and to inactivate pollens, bacteria, viruses, and the like. Note that the discharges may be performed such that the electric charge of one of the discharge generated by the discharge element 38 and the discharge generated by the discharge element 46 is positive and the other is negative. When the discharges are performed as described above, particles of the fine water and the spray agent can be easily mixed by being attracted to each other at the time of mixing the fine water and the atomized medicinal agent. In addition, the discharge may be performed such that an amount of positive charge and an amount of negative charge are equal to each other. As a result, the charge of the whole mixed spray agent S is neutralized, it is therefore possible to prevent the sprayed spray agent S from being electrically sucked to or repelled from the surface of the skin, thereby preventing the permeability and the like from being impaired.

### Second embodiment

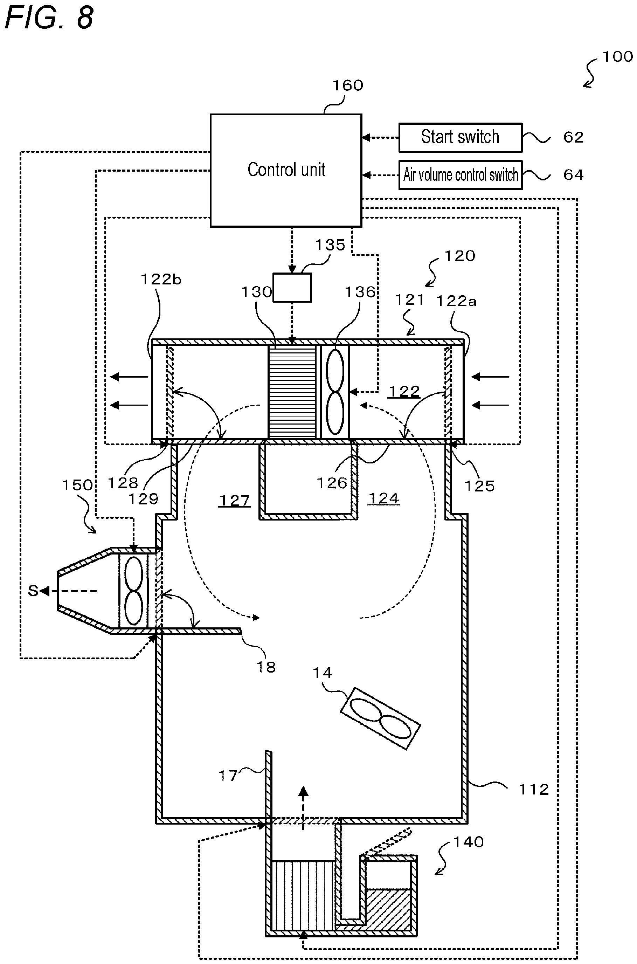

Next, a second embodiment of the present disclosure will be described. FIG. 8 is a configuration diagram schematically illustrating a configuration of a spray device 100 of the second embodiment. The spray device 100 includes a mixing tank 112, a fine water supply unit 120, a medicinal agent atomization unit 140, a spray unit 150, and a control unit 160. The fine water supply unit 120 includes an air passage 121, a fine water generation cartridge 130, an energization circuit 135, and a supply fan 136, and each configuration except the air passage 121 is the same as the configuration of the first embodiment; therefore, the description thereof is omitted.

The air passage 121 includes a main passage 122, a first communication passage 124, and a second communication passage 127, and is provided with a first switching part 125 and a second switching part 128. The main passage 122 is a tubular passage whose both ends are opened. In the main passage 122, the supply fan 136 and the fine water generation cartridge 130 are provided in this order from a first opening 122a on one side toward a second opening 122b on the other side. The first communication passage 124 and the second communication passage 127 communicate the main passage 122 with the mixing tank 112. The first communication passage 124 is connected to the main passage 122 on the first opening 122a side with respect to the supply fan 136 and extends into the mixing tank 112. The second communication passage 127 is connected to the main passage 122 on the second opening 122b side with respect to the fine water generation cartridge 130 and extends into the mixing tank 112.

The first switching part 125 includes a switching plate 126 that operates by being driven by a motor (not illustrated), and the second switching part 128 includes a switching plate 129 that operates by being driven by a motor (not illustrated). As indicated by the solid lines in FIG. 8, when the switching plate 126 is at a normal position (initial position), the first switching part 125 closes (blocks) the first communication passage 124 and opens the first opening 122a side to allow air to flow through the first opening 122a. Further, when the switching plate 129 is at a normal position, the second switching part 128 closes (blocks) the second communication passage 127 and opens the second opening 122b side to allow air to flow through the second opening 122b. In this state, the mixing tank 112 is blocked from the main passage 122 and thereby sealed; therefore, this state is referred to as a sealed state (blocked state).

On the other hand, as indicated by the dotted lines in FIG. 8, when driving of the motor causes the switching plate 126 to operate and move to an operation position where the switching plate 126 is rotated by 90 degrees, the first switching part 125 opens the first communication passage 124 and closes the first opening 122a side to block the flow of air through the first opening 122a. Furthermore, when driving of the motor causes the second switching plate 129 to operate and move to an operation position where the switching plate 129 is rotated by 90 degrees, the second switching part 128 opens the second communication passage 127 and closes the second opening 122b side to block the flow of air through the second opening 122b. In this state, the mixing tank 112 and the main passage 122 communicate with each other through the first communication passage 124 and the second communication passage 127, so that air can circulate through the mixing tank 112 and the main passage 122; therefore, this state is referred to as a circulation state (communication state).

The spray device 100 configured as described above, the moisture absorption control and the moisture release control (supply into the mixing tank 12) of the fine water are performed as follows. In the moisture absorption control, the control unit 160 establishes the above-described sealed state and activates the supply fan 136 while energization to the fine water generation cartridge 130 is turned off, thereby causing moisture to adsorb to the conductive polymer film 34b. As a result, the temperature of conductive polymer film 34b decreases, thereby promoting adsorption of moisture. Note that the rotation direction of the supply fan 136 may be, for example, a direction in which air flows from the first opening 122a to the second opening 122b (see the solid arrows in FIG. 8).

Furthermore, in the moisture release control, the control unit 160 establishes the above-described circulation state and activates the supply fan 136 while energization to the fine water generation cartridge 130 is turned on, thereby causing fine water to be released from the conductive polymer film 34b and supplying the fine water into the mixing tank 112. Note that, the rotation direction of the supply fan 136 may be the same direction as in the moisture absorption control, and the air sent from the supply fan 136 circulates such that the air sent from the supply fan 136 flows from the second communication passage 127 into the mixing tank 112 through the fine water generation cartridge 130 and then returns to the main passage 122 through the first communication passage 124 (see the dotted arrows in FIG. 8). As a result, since the fine water is supplied from the fine water generation cartridge 130 into the mixing tank 112 by using the circulating air without causing new air to flow in, the number of particles of the fine water in the mixing tank 112 increases, so that a high concentration environment of the fine water can be established. As a result, it is possible to spray the spray agent in which the fine water is sufficiently mixed; therefore, it is possible to further improve the permeability and the moisture retaining property of the active ingredient.

In the first embodiment and the second embodiment, a humidity sensor may be provided in the mixing tanks 12 and 112, and the supply time, the mixing time, and the spray time may be adjusted, or the air volume of each fan may be changed, by using the humidity detected by the humidity sensor as an alternative characteristic for the number of fine water particles.

### Third embodiment

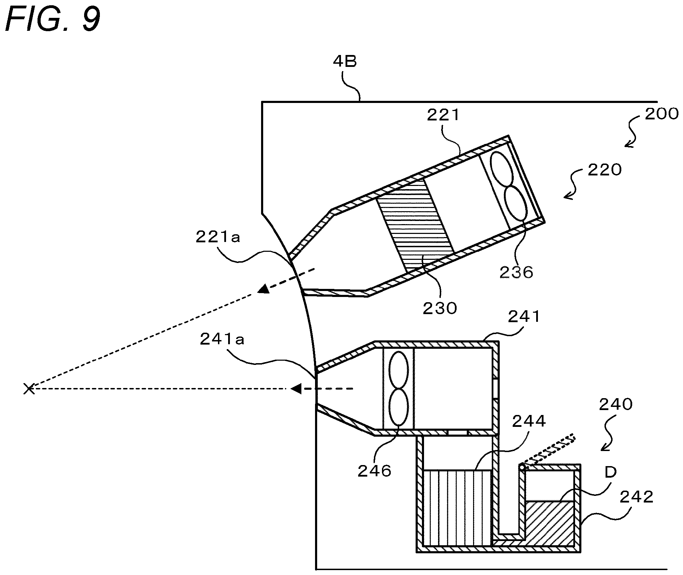

Next, a third embodiment of the present disclosure will be described. FIG. 9 is a configuration diagram schematically illustrating a configuration of a spray device 200 of the third embodiment. The spray device 200 is disposed in a discharge head 4B and includes a fine water supply unit 220, a medicinal agent atomization unit 240, and a control unit (not illustrated). This spray device 200 is configured not to include the mixing tank 12 or the spray unit 50 and to separately spray the fine water and the atomized medicinal agent without mixing the fine water and the atomized medicinal agent.

Similarly to the fine water supply unit 20, the fine water supply unit 220 includes a fine water generation cartridge 230 and a supply fan 236 in a duct 221, and discharges the fine water from the fine water discharge port 221a toward a treatment target place (mark × in the drawing). Similarly to the medicinal agent atomization unit 40, the medicinal agent atomization unit 240 includes a medicinal agent tank 42 and an atomizer 44. In addition, the medicinal agent atomization unit 240 includes: a duct 241 to which a medicinal agent atomized by the atomizer 44 is supplied; and a fan 246 to eject the atomized medicinal agent (air) in the duct 241 from the medicinal agent discharge port 241a, and the atomized medicinal agent is discharged from the medicinal agent discharge port 241a toward the treatment target place.

Because the spray device 200 of the third embodiment can separately discharge the fine water and the atomized medicinal agent, the fine water and the atomized medicinal agent can be separately sprayed to skin or hair. Therefore, it is possible to spray the fine water first to form water routes and then spray the atomized medicinal agent, and, conversely, it is possible to spray the fine water after spraying the atomized medicinal agent. These methods may be switched depending on the difference in medicinal components and the purpose of the treatment of skin or hair. In addition, since the atomized medicinal agent can be sprayed during the moisture absorption control of the fine water, the cosmetic treatment can be efficiently performed.

The fine water supply unit 20, 120, or 220 of each embodiment may include a temperature control cartridge (heat exchanger) in addition to the fine water generation cartridge 30, 130, or 230 and the supply fan 36, 136, or 236. The temperature control cartridge is formed of a metal material in, for example, a corrugated plate shape, a honeycomb shape, a spiral shape, or the like so that the temperature control cartridge has a large heat capacity and high heat exchange efficiency. Then, the air containing the fine water released from the fine water generation cartridge 30, 130, or 230 during the moisture release control may be supplied into the mixing tank 12 or 112 or discharged from the fine water discharge port 221a after passing through the temperature control cartridge. In this way, because the air whose temperature has increased in the fine water generation cartridge 30, 130, or 230 is cooled to near room temperature when passing through the temperature control cartridge, the fine water can be appropriately supplied and discharged without increasing the temperature.

The correspondence relationship between the main elements of the embodiments and the main elements of the present disclosure described in the section of SOLUTIONS TO PROBLEMS will be described. In the embodiment, S270 and S280 of the spray process correspond to the step of spraying. Furthermore, S240 and S250 correspond to the step of mixing. Furthermore, the spray device 10 (10B, 100, 200) corresponds to the "spray device", the medicinal agent atomization unit 40 (40B, 140, 240) corresponds to the "atomization device", the fine water generation cartridge 30 (30B, 130, 230) corresponds to the "fine water generator", and the spray unit 50 (150) corresponds to the "spray unit". Furthermore, the mixing tank 12 (112) and the mixing fan 14 correspond to the "mixing unit".

Note that the correspondence relationship between the main elements of the embodiments and the main elements of the present disclosure described in the section of SOLUTIONS TO PROBLEMS is an example for specifically describing forms for carrying out the present disclosure described in the section of SOLUTIONS TO PROBLEMS; therefore, the correspondence relationship does not limit the elements of the present disclosure described in the section of SOLUTIONS TO PROBLEMS. That is, the interpretation of the present disclosure described in the section of SOLUTIONS TO PROBLEMS should be made based on the description in the section, and the embodiments are merely a specific example of the present disclosure described in the section of SOLUTIONS TO PROBLEMS Although the embodiments for carrying out the present disclosure have been described above with reference to the embodiments, the present disclosure is not limited to such embodiments at all, and can be carried out in various forms without departing from the gist of the present disclosure.

### Industrial applicability

The present disclosure can be used for cosmetic treatment in which a spray agent for skin or hair is sprayed.

## Claims

1. A spray agent for skin or hair comprising a mixture,
wherein the mixture includes:
an atomized medicinal agent in which a medicinal agent containing a predetermined active ingredient is atomized; and
fine water that is uncharged and has a particle size of less than or equal to 50 nanometers.

2. A cosmetic treatment method comprising: spraying toward skin or hair, an atomized medicinal agent in which a medicinal agent containing a predetermined active ingredient is atomized, and fine water that is uncharged and has a particle size of less than or equal to 50 nanometers.

3. The cosmetic treatment method according to claim 2, further comprising: mixing the atomized medicinal agent and the fine water,
wherein in the spraying, the spray agent in which the atomized medicinal agent and the fine water are mixed in the mixing is sprayed toward skin or hair.

4. A spray device comprising:
an atomizer that generates an atomized medicinal agent in which a medicinal agent containing a predetermined active ingredient is atomized;
a fine water generator that changes to a moisture absorption state in which moisture in air is made to be adsorbed to a conductive polymer film due to a decrease in temperature, and to a moisture release state in which the moisture adsorbed to the conductive polymer film is made to be released as fine water having a particle size of less than or equal to 50 nanometers due to an increase in temperature; and
a spray unit that sprays the atomized medicinal agent and the fine water toward skin or hair.

5. The spray device according to claim 4, further comprising:
a mixing unit that communicates with the atomizer, the fine water generator, and the spray unit and mixes the atomized medicinal agent and the fine water,
wherein the spray unit sprays a spray agent in which the atomized medicinal agent and the fine water are mixed in the mixing unit.

6. The spray device according to claim 5, further comprising:
a switching part that selects any one of a sealed state in which communication between the fine water generator and the mixing unit is blocked and air outside the mixing unit is supplied to the fine water generator, and a circulation state in which communication is established between the fine water generator and the mixing unit and air inside the mixing unit is supplied to the fine water generator,
wherein the fine water generator enters the moisture absorption state when the switching part is in the sealed state, and the moisture release state when the switching part is in the circulation state, such that a concentration of the fine water in the mixing unit is increased.

7. The cosmetic treatment method according to claim 3, further comprising:
adsorbing moisture to a conductive polymer film having a nanochannel; and
increasing a temperature of the conductive polymer film to release the moisture adsorbed to the conductive polymer film,
wherein in the mixing, the fine water is mixed with the atomized medicinal agent.
